(19) Europäisches Patentamt
European Patent Office
Office européen des brevets

(11) Veröffentlichungsnummer: **0 008 333**
**B1**

(12) **EUROPÄISCHE PATENTSCHRIFT**

(45) Veröffentlichungstag der Patentschrift: **16.02.83**

(21) Anmeldenummer: **79102087.8**

(22) Anmeldetag: **25.06.79**

(51) Int. Cl.³: **C 07 C 33/28,**
A 01 N 53/00,
C 07 C 29/40,
C 07 C 33/48,
C 07 C 43/215,
C 07 C 43/225,
C 07 C 69/743,
C 07 C 93/14,
C 07 C 121/75

# (54) Alpha-Prop-1-inyl-3-phenoxybenzylalkohole, ihre Herstellung und Verwendung als Zwischenprodukte zur Herstellung von Schädlingsbekämpfungsmitteln.

(30) Priorität: **27.06.78 CH 6994/78**
**19.04.79 CH 3681/79**

(43) Veröffentlichungstag der Anmeldung:
**05.03.80 Patentblatt 80/5**

(45) Bekanntmachung des Hinweises auf die Patenterteilung:
**16.02.83 Patentblatt 83/7**

(84) Benannte Vertragsstaaten:
**AT BE CH DE FR GB IT NL**

(56) Entgegenhaltungen:
**DE - A - 2 527 352**
**DE - A - 2 615 435**
**DE - A - 2 626 018**
**DE - A - 2 727 909**

(73) Patentinhaber: **CIBA-GEIGY AG**
**Patentabteilung Postfach**
**CH-4002 Basel (CH)**

(72) Erfinder: **Farooq, Saleem, Dr.**
**Im Schaiengarten 2**
**CH-4107 Ettingen (CH)**
Erfinder: **Ackermann, Peter, Dr.**
**Reichensteinerstrasse 12**
**CH-4153 Reinach (CH)**
Erfinder: **Drabek, Jozef, Dr.**
**Benkenstrasse 12**
**CH-4104 Oberwil (CH)**
Erfinder: **Gsell, Laurenz, Dr.**
**Maiengasse 56**
**CH-4056 Basel (CH)**
Erfinder: **Kristiansen, Odd, Dr.**
**Delligrabenstrasse 7**
**CH-4313 Möhlin (CH)**
Erfinder: **Wehrli, Rudolf, Dr.**
**Dianastrasse 2**
**CH-4310 Rheinfelden (CH)**

(74) Vertreter: **Zumstein, Fritz sen., Dr. et al,**
**Bräuhausstrasse 4**
**D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England

EP 0 008 333 B1

# Alpha-Prop-1-inyl-3-phenoxybenzylalkohole, ihre Herstellung und Verwendung als Zwischenprodukte zur Herstellung von Schädlingsbekämpfungsmitteln

Die vorliegende Erfindung betrifft $\alpha$-Prop-1-ynyl-3-phenoxybenzylalkohole, Verfahren zur ihrer Herstellung und ihre Verwendung als Zwischenprodukte zur Synthese von Verbindungen, welche sich zur Schädlingsbekämpfung eignen.

Die $\alpha$-Prop-1-ynyl-3-phenoxybenzylalkohole haben die Formel

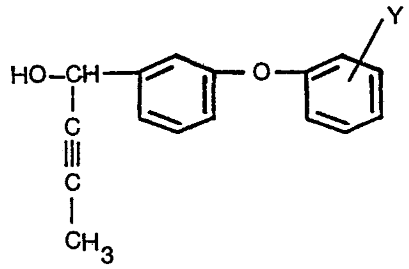

worin Y Wasserstoff, Halogen, $C_1$—$C_4$-Alkyl, $C_1$—$C_3$-Alkoxy, Cyano oder Dimethylamino bedeutet.

Unter Halogen ist dabei Fluor, Chlor, Brom oder Jod, insbesondere aber Fluor, Chlor oder Brom, zu verstehen.

Die Alkyl- und Alkoxygruppen sind: Methyl, Methoxy, Aethyl, Aethoxy, Propyl, Propoxy, Isopropyl, Isopropoxy, n-, i-, sek.- und tert.-Butyl.

Insbesondere geeignet als Zwischenprodukte sind Verbindungen der Formel I, worin Y Wasserstoff, Fluor, Chlor, Brom, Methyl oder Methoxy bedeutet.

Die Verbindungen der Formel I können analog bekannten Methoden z.B. wie folgt hergestellt werden:

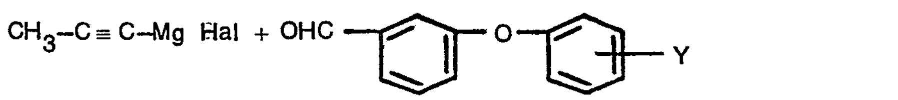

(II) (III) I

In der Formel III hat Y die für die Formel I angegebene Bedeutung und in der Formel II steht Hal für ein Halogenatom, insbesondere für Chlor oder Brom.

Das Verfahren wird bei einer Reaktionstemperatur won —10 bis 100°C, vorzugsweise bei 0 bis 80°C, im allgemeinen bei Normaldruck und in einem inerten Lösungs- oder Verdünnungsmittel durchgeführt. Als Lösungs- oder Verdünnungsmittel eignen sich vor allem Aether wie Diäthyläther, Tetrahydrofuran und Dioxan. Die Ausgangsstoffe der Formeln II und III sind bekannt oder können analog bekannten Methoden hergestellt werden.

Die Verbindungen der Formel I eignen sich vor allem als Zwischenprodukte für die Herstellung von Pyrethroiden, wie sie in der europäischen Anmeldung No. 79 102 085.2, publiziert unter der Nummer 8332, beschrieben sind und welche sich zur Bekämpfung von Schädlingen, insbesondere von Insekten und Vertretern der Ordnung Akarina, eignen.

## Beispiel 1

a) Herstellung von $\alpha$-Prop-1-ynyl-3-phenoxybenzylalkohol

Zu einer Lösung von 8 g Methylacetylen in 100 ml Tetrahydrofuran wird bei 0°C eine frisch aus 4 g Magnesium, 20 g Aethylbromid in 20 ml Tetrahydrofuran hergestellte Grignardlösung langsam zugegeben und während 15 Minuten unter Argon gerührt. Zu diesem Gemisch wird eine Lösung von 27 g 3-Phenoxybenzaldehye in 100 ml Tetrahydrofuran bei o bis 5°C zugetropft.

Nach 14 stündigem Rühren bei Raumtemperatur wird das Reaktionsgemisch mit 50 g Eis auf 0°C abgekühlt, langsam mit 25 ml conc. Salzsäure versetzt und mit Aether extrahiert. Der Aether-Extrakt wird zweimal mit Wasser un zweimal mit gesättigter Kochsalzlösung gewaschen, über Natriumsulfat getrocknet, filtriert und eingeengt.

Das Produkt wird mit Essigester/Hexan (1:4) als Eluiertmittel über Silikagel chromatographiert. Man erhält die Verbindung der Formel

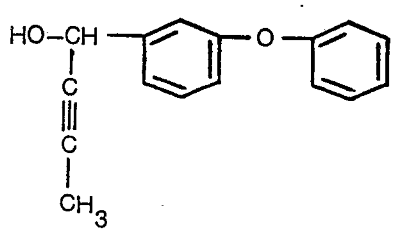

mit einem Brechungsindex von $n_D^{20°} = 1,5898$.

Auf analoge Weise werden auch folgende Verbindungen hergestellt:

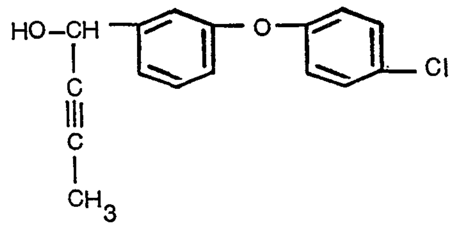

$n_D^{20°} = 1,5844$

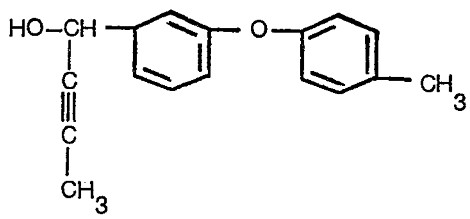

$n_D^{20°} = 1,5710$

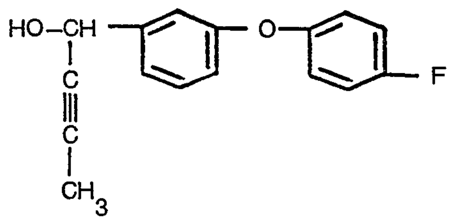

$n_D^{20°} = 1,5669$

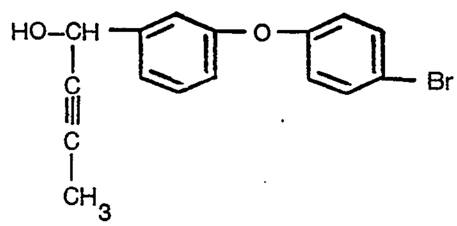

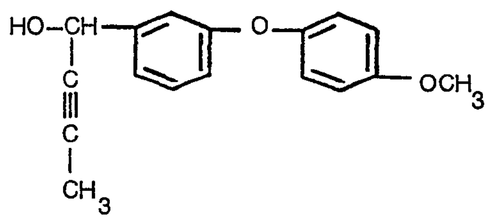

b) Herstellung von α-Prop-1-ynyl-3-phenoxybenzyl-2,2-dimethyl-3-(2′,2′-dichlorvinyl)cyclopropan-1-carboxylat

Zu einer eisgekühlte Lösung von 3,82 g 2,2-Dimethyl-3-(2′,2′-dichlorvinyl)-cyclopropancarbonsäurechlorid und 1,8 ml Pyridin in 50 ml Toluol wird eine Lösung von 4 g α-Prop-1-ynyl-3-phenoxybenzylalkohol in 20 ml Toluol zugetropft.

Das Reaktionsgemisch wird 14 Stunden bei Raumtemperatur gerührt und dann mit Aether versetzt. Der Aetherextrakt wird einmal mit Wasser, einmal mit 2n-Salzsäure und dreimal mit gesättigter Kochsalzlösung gewaschen, anschliessend über Natriumsulfat getrocknet, filtriert und eingeengt. Das Produkt wird mit Aether/Hexan (1:3) als Eluiermittel über Silikagel chromatographiert.

Man erhält die Verbindung der Formel

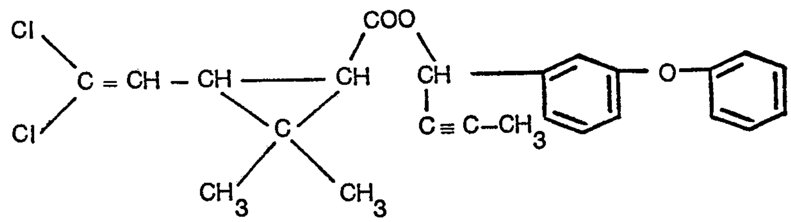

als diastereomeres Gemisch mit einem Brechungsindex von $n_D^{20°} = 1{,}5700$.

**Patentansprüche für die Vertragsstaaten: BE CH DE FR GB IT NL**

1. Ein $\alpha$-Prop-1-ynyl-3-phenoxybenzylalkohol der Formel

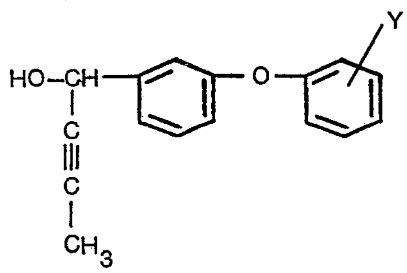

worin Y Wasserstoff, Halogen, $C_1$—$C_4$-Alkyl, $C_1$—$C_3$-Alkoxy, Cyano oder Dimethylamino bedeutet.

2. Eine Verbindung gemäss Anspruch 1, worin Y Wasserstoff, Fluor, Chlor, Brom, Methoxy oder Methyl bedeutet.

3. Die Verbindung gemäss Anspruch 2 der Formel

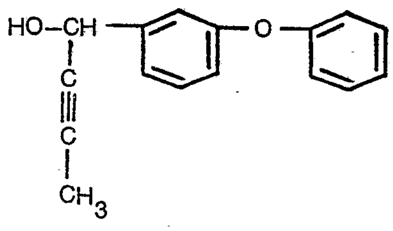

4. Die Verbindung gemäss Anspruch 2 der Formel

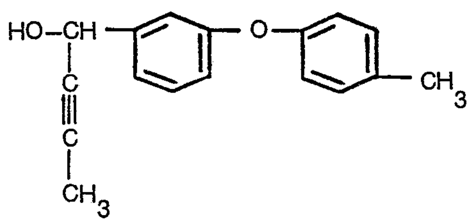

5. Die Verbindung gemäss Anspruch 2 der Formel

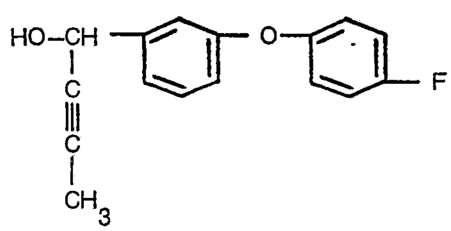

6. Verwendung eines Alkohols gemäss Anspruch 1 zur Herstellung von Pyrethroiden, welche sich zur Bekämpfung von Schädlingen, insbesondere von Insekten oder Vertretern der Ordnung Akarina, eignen.

7. Verfahren zur Herstellung eines Alkohols gemäss Anspruch 1, dadurch gekennzeichnet, dass man eine Verbindung der Formel

$$CH_3C \equiv C—Mg\ Hal$$

mit einer Verbindung der Formel

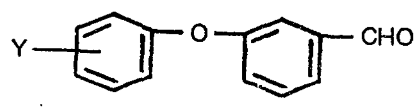

umsetzt, worin Y die im Anspruch 1 angegebene Bedeutung hat und Hal für ein Halogenatom steht.

**Patentanspruch für den Vertragsstaat: AT**

Ein Verfahren zur Herstellung eines Alkohols der allgemeinen Formel

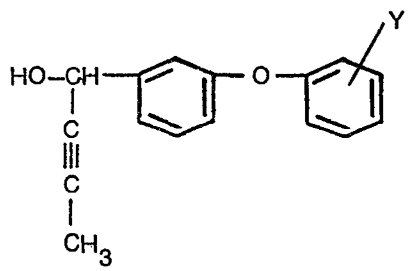

worin Y Wasserstoff, Halogen, $C_1$—$C_4$ Alkyl, $C_1$—$C_3$-Alkoxy, Cyano oder Dimethylamino bedeutet, dadurch gekennzeichnet, dass man eine Verbindung der Formel

$$CH_3C \equiv C—MgHal$$

mit einer Verbindung der Formel

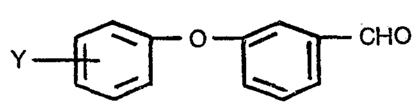

umsetzt, worin Y die oben angegebene Bedeutung hat und Hal für ein Halogenatom steht.

**Claims for the Contracting States: BE CH DE FR GB IT NL**

1. A $\alpha$-prop-1-ynyl-3-phenoxybenzyl alcohol of the formula

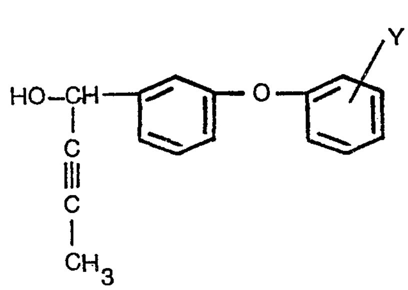

wherein Y represents hydrogen, halogen, $C_1$—$C_4$alkyl, $C_1$—$C_3$alkoxy, cyano or dimethylamino.

2. A compound according to claim 1, wherein Y represents hydrogen, fluorine, chlorine, bromine, methoxy or methyl.

3. The compound according to claim 2 of the formula

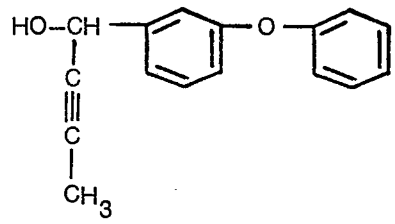

4. The compound according to claim 2 of the formula

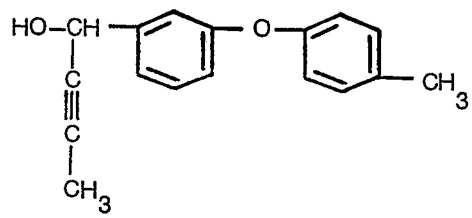

5. The compound according to claim 2 of the formula

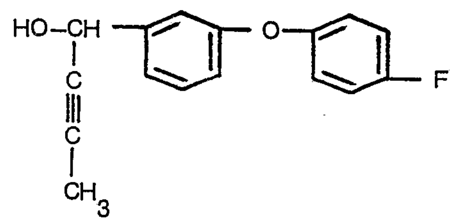

6. The use of an alcohol according to claim 1 for the manufacture of pyrethroids which are suitable for controlling pests, in particular insects or representatives of the order Acarina.

7. A process for the manufacture of an alcohol according to claim 1, which comprises reacting a compound of the formula

$$CH_3C \equiv C\text{—}MgHal$$

with a compound of the formula

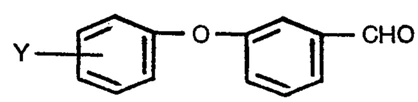

wherein Y is as defined in claim 1 and Hal represents a halogen atom.

**Claim for the Contracting State: AT**

A process for the manufacture of an alcohol of the general formula

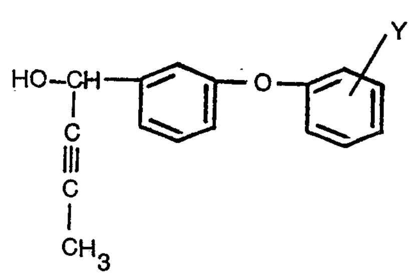

wherein Y is hydrogen, halogen, $C_1$—$C_4$alkyl, $C_1$—$C_3$alkoxy, cyano or dimethylamino, which process comprises reacting a compound of the formula

$$CH_3C \equiv C\text{—}MgHal$$

with a compound of the formula

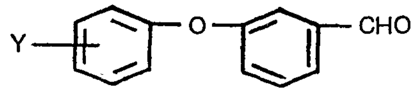

wherein Y has the above meaning and Hal is a halogen atom.

**Revendications pour les Etats contractants: BE CH DE FR GB IT NL**

1. Alcool $\alpha$-prop-1-ynyl-3-phénoxybenzylique de formule

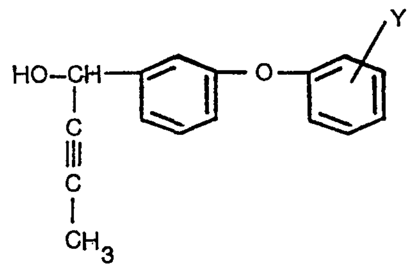

où Y représente un hydrogène, un halogène, un alcoyle en $C_1$ à $C_4$, un alcoxy en $C_1$ à $C_3$, un cyano ou un diméthylamino.

2. Composé selon la revendication 1, où Y représente un hydrogène, un fluor, un chlore, un brome, un méthoxy ou un méthyle.

3. Composé selon la revendication 2 de formule

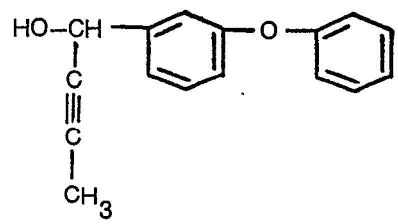

4. Composé selon la revendication 2 de formule

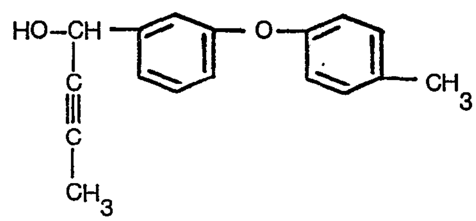

5. Composé selon la revendication 2 de formule

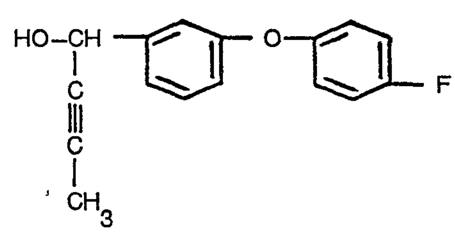

6. Application d'un alcool selon la revendication 1 à la préparation de pyréthroïdes appropriés à la lutte contre les parasites, en particulier les insectes ou les représentants de l'ordre des acariens.

7. Procédé de préparation d'un alcool selon la revendication 1, caractérisé en ce qu'on fait réagir un composé de formule

$$CH_3C\equiv C—Mg\ Hal$$

avec un composé de formule

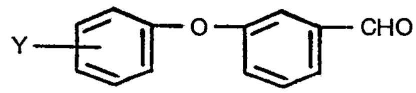

où Y a la signification donnée dans la revendication 1 et Hal représente un atome d'halogène.

**Revendication pour l'Etat contractant: AT**

Procédé de préparation d'un alcool de formule générale

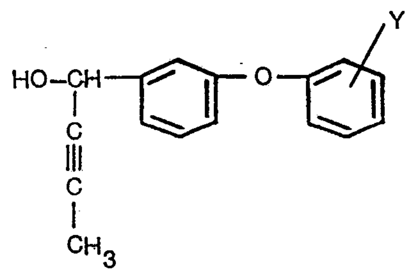

où Y représente un hydrogène, un halogène, un alcoyle en $C_1$ à $C_4$, un alcoxy en $C_1$ à $C_3$, un cyano ou un diméthylamino, caractérisé en ce qu'on fait réagir un composé de formule

$$CH_3C \equiv C—MgHal$$

avec un composé de formule

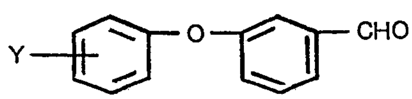

où Y a la signification donnée ci-dessus et Hal représente un atome d'halogène.